# DEMANDE DE BREVET EUROPEEN

(11) **EP 2 550 941 A1**
(43) Date de publication de la demande: **30.01.2013**
(21) Numéro de dépôt: 12177949.0
(22) Date de dépôt: 26.07.2012
(51) Int. Cl.: A61F 2/08, A61B 17/04

(54) **Ensemble de fixation pour fixer un greffon orthopedique sur une surface osseuse et procede de fixation d'un tel ensemble de fixation**

(30) Priorité: 28.07.2011 FR 1156911
(71) Demandeur: Amplitude, 26000 Valence (FR); Cousin Biotech, 59117 Wervicq Sud (FR)
(72) Inventeur: Jallabert, Olivier, 26000 Valence (FR); Geais, Laurent, 26100 Romans (FR); Noel, Stéphane, 59496 Hantay (FR); Noel, Lam-Xé, 59496 Hantay (FR)
(74) Mandataire: Chevalier, Renaud Philippe

(57) **Abrégé**

Cet ensemble de fixation (1) comprend :
- un élément de fixation (2) pour fixer un greffon orthopédique sur une surface osseuse ;
- au moins un fil (3, 4) à deux ou plus portions d'extrémité et lié à l'élément de fixation (2) de façon à réaliser un mouvement respectif de l'élément de fixation (2).

Au moins deux des portions d'extrémité sont solidarisées au moyen d'au moins une soudure (5) en un matériau plastique.

## Description

La présente invention concerne un ensemble de fixation pour fixer sur une surface osseuse un greffon orthopédique. Par ailleurs, la présente invention concerne un procédé de fixation mettant en oeuvre un tel ensemble de fixation.

La présente invention trouve notamment application dans le domaine de l'orthopédie et de la chirurgie orthopédique, en particulier pour la reconstruction des ligaments ou des tendons. Plus particulièrement, la présente invention peut être appliquée à la reconstruction d'un ligament croisé ou d'un tendon.

EP2191791A2 décrit un ensemble de fixation comprenant une platine pour fixer un greffon de ligament croisé antérieur contre la corticale d'un fémur. Pour réaliser des mouvements de la platine, l'ensemble de fixation de EP2191791A2 comprend deux fils de traction qui présentent chacun deux portions d'extrémité. Chaque fil de traction est passé à travers un trou respectif de la platine. Pour passer et guider l'ensemble de fixation avec le greffon orthopédique à travers un os, un opérateur utilise une broche ou une aiguille. Préalablement, l'opérateur doit insérer chaque extrémité d'un fil de traction à travers le chas de la broche. Puis, l'opérateur passe la broche à travers chaque passage osseux. Lorsque la platine a traversé les passages osseux, l'opérateur peut exercer des tractions sur les fils de traction, de façon à faire pivoter la platine et ainsi la plaquer contre la surface osseuse.

Cependant, l'introduction des fils dans le chas de la broche est particulièrement difficile à réaliser, car le chas est très étroit relativement au diamètre des fils de traction. Par exemple, le chas peut avoir une largeur d'environ 1 mm et une longueur d'environ 6 mm, pour insérer des fils de traction dont le diamètre est généralement compris entre 0.5 mm et 1,0 mm.

La présente invention vise notamment à résoudre, en tout ou partie, les problèmes mentionnés ci-avant.

A cet effet, l'invention a pour objet un ensemble de fixation, pour fixer sur une surface osseuse un greffon orthopédique, l'ensemble de fixation comprenant :
- au moins un élément de fixation adapté pour fixer sur une surface osseuse une partie du greffon orthopédique;
- au moins un fil qui présente au moins deux portions d'extrémité et qui est lié à l'élément de fixation de sorte qu'une traction exercée sur ledit fil réalise un mouvement respectif de l'élément de fixation;
l'ensemble de fixation étant **caractérisé en ce qu'**au moins deux desdites portions d'extrémité sont solidarisées au moyen d'au moins une soudure formée par au moins un matériau plastique de synthèse.

En d'autres termes, l'élément de fixation est en liaison avec un ou plusieurs fils de traction, dont toutes ou certaines extrémités sont soudées entre elles de façon à former un ou plusieurs faisceau(x) comprenant chacun au moins deux extrémités soudées. De plus, chaque portion d'extrémité est soudée à au moins une autre portion d'extrémité, ces portions d'extrémité appartenant à un même fil ou à deux fils distincts.

Ainsi, un tel ensemble de fixation facilite le passage des fils dans le chas d'une broche ou d'une aiguille de guidage, par exemple en vue de faire traverser le greffon orthopédique dans un ou plusieurs passage(s) osseux. De plus, un tel ensemble de fixation évite qu'un fil de traction ne se détache de l'élément de fixation, en particulier au cours de l'opération orthopédique.

Dans la présente demande, le terme « élément de fixation » désigne tout élément adapté pour fixer sur une surface osseuse une partie d'un greffon orthopédique, généralement une extrémité d'un greffon orthopédique. Cette fonction de fixation peut être remplie par des éléments de fixation présentant des structures ou des formes très diverses. Par exemple, il peut s'agir :
- d'une platine plaquée contre la corticale d'un fémur;
- d'un dispositif dit impacté, qui est emmanché à force dans un passage osseux;
- d'un dispositif dit vissé, qui comprend une vis traversant un passage osseux;
- d'un dispositif à expansion, comprenant un composant expansé dans un passage osseux par l'insertion d'une tige ou d'une vis;
- d'un dispositif dit à bascule, qui est introduit dans une cavité osseuse et que l'on fait basculer pour l'ancrer à l'intérieur le passage osseux.

Dans la présente demande, le terme « fil » désigne un élément allongé et souple. Un tel fil peut être réalisé en un ou plusieurs matériau(x), naturel(s) et/ou synthétique(s). Un tel fil peut comprendre une seule fibre ou plusieurs fibres, par exemple plusieurs brins tressés.

Dans la présente demande, le terme « greffon orthopédique » recouvre tout type de greffon orthopédique, en matériau(x) naturel(s) et/ou synthétique(s), le greffon orthopédique pouvant remplir une fonction ligamentaire, tendineuse ou osseuse. Par exemple, un tel greffon orthopédique peut réparer ou remplacer un ligament croisé, un tendon d'une coiffe des rotateurs, tel qu'un tendon susépineux, ou un tissu osseux tel qu'une butée d'épaule pour un transfert de coracoïde en avant de la glène. Pour remplacer un ligament croisé, le greffon orthopédique peut être par exemple formé par un tendon préalablement prélevé sur le patient.

Selon un mode de réalisation de l'invention, au moins un fil est composé totalement ou partiellement par ledit matériau plastique de synthèse.

Ainsi, une telle soudure autogène évite d'apporter un composant supplémentaire lors du soudage des portions d'extrémité, ce qui simplifie ce soudage.

Selon une variante de l'invention, ledit matériau plastique de synthèse est apposé sur les portions d'extrémité.

Ainsi, une telle soudure hétérogène, c'est-à-dire avec apport de matière en cours du soudage, permet de réaliser des soudures dont la résistance mécanique est de fiabilité particulièrement élevée.

Selon un mode de réalisation de l'invention, le matériau plastique de synthèse est sélectionné dans le groupe constitué du polyester, polypropylène, polyéthylène haute ténacité, polyamide, PLLA, ou copolymère d'acide lactique et glycolique.

Ainsi, un tel matériau est facile à mettre en oeuvre, notamment car il présente une température de ramollissement ou de fusion relativement basse. De plus, un tel matériau peut être biocompatible.

Selon un mode de réalisation de l'invention, lesdites au moins deux portions d'extrémité sont substantiellement parallèles et ont des parties terminales adjacentes.

Ainsi, un faisceau formé par de telles portions d'extrémité est relativement compact, ce qui simplifie son passage à travers le chas d'une broche de guidage.

Selon un mode de réalisation de l'invention, l'ensemble de fixation comprend au moins deux fils, chacun desdits fils est lié à l'élément de fixation de sorte qu'une traction exercée sur un fil respectif réalise un mouvement respectif de l'élément de fixation, et au moins quatre portions d'extrémité sont solidarisées ensemble.

Ainsi, une seule étape est nécessaire pour introduire un faisceau multifils dans le chas d'une broche de guidage.

Selon un mode de réalisation de l'invention, lesdites au moins quatre portions d'extrémité sont deux à deux juxtaposées de sorte qu'elles forment un faisceau plat, le faisceau plat ayant de préférence une épaisseur inférieure à 1 mm et une largeur inférieure à 6 mm.

Ainsi, un tel faisceau plat peut être introduit et enfilé dans un chas relativement étroit.

Selon une variante de l'invention, un ensemble de fixation comprend deux fils de traction présentant chacun deux portions d'extrémité, les quatre portions d'extrémité étant solidarisées par groupe de deux portions d'extrémité de façon à former deux faisceaux distincts.

Ainsi, on peut séparer chaque faisceau indépendamment de l'autre faisceau, par exemple à des instants différents, en cas de besoin.

Selon un mode de réalisation de l'invention, au moins deux fils ont des couleurs différentes, par exemple un fil blanc et un fil vert.

Ainsi, l'opérateur peut facilement distinguer deux fils de traction afin de réaliser de manière certaine tel ou tel mouvement spécifique de l'élément de fixation.

Selon un mode de réalisation de l'invention, ladite au moins une soudure est conçue pour présenter un effort de séparation de deux portions d'extrémité qui est inférieur à 12 newtons, de préférence à 8 newtons, de préférence encore à 6 newtons.

Ainsi, la séparation des portions d'extrémité peut être réalisée manuellement par un opérateur, tel qu'un chirurgien. L'effort de séparation est produit en tirant les deux portions d'extrémité globalement suivant des sens opposés, de façon à rompre la ou chaque soudure.

Selon un mode de réalisation de l'invention, ladite au moins une soudure est conçue pour résister à un effort de séparation de deux portions d'extrémité qui est supérieur à 0,8 newtons, de préférence à 1,0 newtons.

Ainsi, une telle résistance de la ou des soudure(s) évite une séparation intempestive des portions d'extrémité. L'effort de séparation est produit en tirant les deux portions d'extrémité globalement suivant des sens opposés, de façon à rompre la ou chaque soudure.

Selon un mode de réalisation de l'invention, ladite au moins une soudure est réalisée par soudage thermique par effet Joule, par laser, par haute fréquence ou par ultrasons ou par soudage à froid au moyen de solvants.

Ainsi, de telles soudures sont mécaniquement résistantes et rapides à réaliser.

Selon un mode de réalisation de l'invention, lesdites au moins deux portions d'extrémité sont solidarisées sur une longueur supérieure à 1 mm, de préférence à 3 mm.

Ainsi, une telle longueur de soudure permet d'éviter toute séparation intempestive des portions d'extrémité, en fonction du matériau et de l'épaisseur de soudure.

Selon un mode de réalisation de l'invention, au moins deux portions d'extrémité respectives présentent des parties terminales qui sont exemptes de soudure et qui ont de préférence une longueur comprise entre 0,1 mm et 15,0 mm, ladite au moins une soudure étant formée en amont de chacune desdites parties terminales.

Dans la présente demande, le terme « partie terminale » désigne, au sein d'une portion d'extrémité, la partie qui se trouve tout à fait à l'extrémité du fil.

Ainsi, de telles parties terminales « libres » facilitent la prise par l'opérateur de chaque portion d'extrémité en vue de les séparer.

Selon un mode de réalisation de l'invention, au moins deux portions d'extrémité respectives présentent des parties terminales solidarisées par ladite au moins une soudure.

Ainsi, des parties terminales solidarisées par soudure permettent de faciliter l'introduction de ces parties terminales, donc le passage des fils, à travers le chas d'une broche de guidage.

Selon un mode de réalisation de l'invention, une portion d'extrémité présente une dureté superficielle supérieure à la dureté du reste du fil respectif.

Ainsi, une telle dureté superficielle permet de faciliter le passage des portions d'extrémité à travers le chas d'une broche de guidage.

Selon un mode de réalisation de l'invention, ledit au moins fil a un diamètre inférieur à 1,0 mm, de préférence inférieur à 0,8 mm.

Dans la présente demande, le terme « diamètre » désigne une dimension d'un fil mesurée dans un plan transversal à la direction longitudinale de ce fil.

Ainsi, un tel diamètre de fil permet de faciliter le passage du fil à travers le chas d'une broche de guidage, tout en conférant au fil une résistance mécanique appropriée.

Selon un mode de réalisation de l'invention, l'élément de fixation présente deux trous distants, le ou chaque fil passant dans un trou de façon à former, avec les portions d'extrémité correspondantes, une boucle liant ledit fil à l'élément de fixation, l'élément de fixation étant par exemple une platine destinée à fixer un greffon orthopédique formant un ligament croisé.

Ainsi, chaque fil de traction peut être lié de manière simple à l'élément de fixation.

Selon un mode de réalisation de l'invention, l'élément de fixation est une platine destinée à fixer un greffon orthopédique formant un ligament croisé.

Ainsi, un tel ensemble de fixation est adapté pour l'orthopédie du ligament croisé.

Par ailleurs, la présente invention a pour objet un procédé de fixation, pour fixer sur une surface osseuse un greffon orthopédique qui est destiné à traverser au moins un passage osseux, le procédé de fixation comprenant les étapes :
- mettre en oeuvre un ensemble de fixation selon l'invention;
- lier ledit au moins un fil à l'élément de fixation de sorte qu'une traction exercée sur un fil respectif réalise un mouvement respectif de l'élément de fixation; et
- solidariser au moins deux desdites portions d'extrémité au moyen d'au moins une soudure formée par au moins un matériau plastique de synthèse, ladite au moins une soudure étant est réalisée par un procédé de soudage sélectionné dans le groupe constitué du soudage thermique par effet Joule, par laser, par haute fréquence ou par ultrasons et du soudage à froid au moyen de solvants.

Ainsi, un tel procédé permet d'assembler un ensemble de fixation conforme à l'invention.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue de face d'un ensemble de fixation conforme à l'invention;
- la figure 2 est une vue du détail II à la figure 1
- la figure 3 est une vue du détail III à la figure 1;
- la figure 4 est une vue schématique en perspective de l'ensemble de fixation de la figure 1 en cours de service;
- la figure 5 est une vue schématique en perspective et à plus grande échelle d'une partie de l'ensemble de fixation de la figure 4;
- la figure 6 est une vue similaire à la figure 3 d'une partie d'un ensemble de fixation conforme à un deuxième mode de réalisation de l'invention; et
- la figure 7 est une vue similaire à la figure 3 d'une partie d'un ensemble de fixation conforme à un troisième mode de réalisation de l'invention.

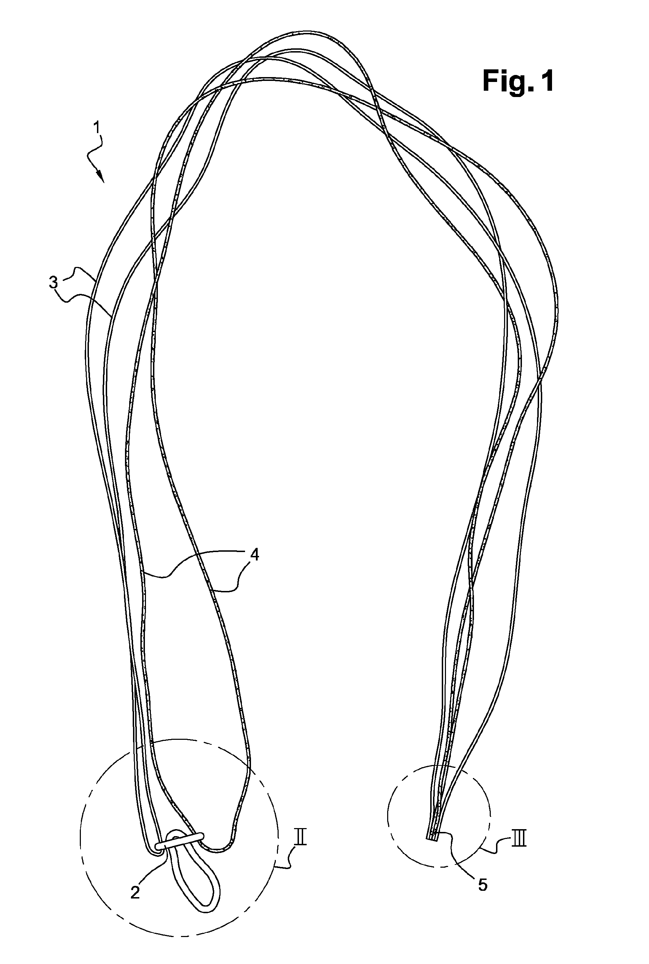

La figure 1 illustre un ensemble de fixation 1, qui a notamment pour fonction de fixer sur une surface osseuse un greffon orthopédique.

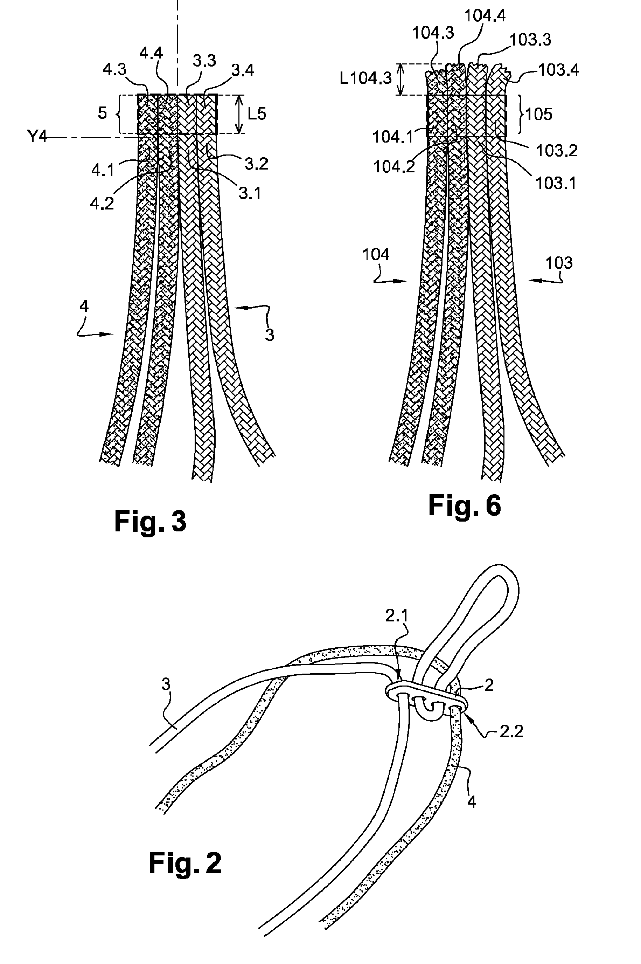

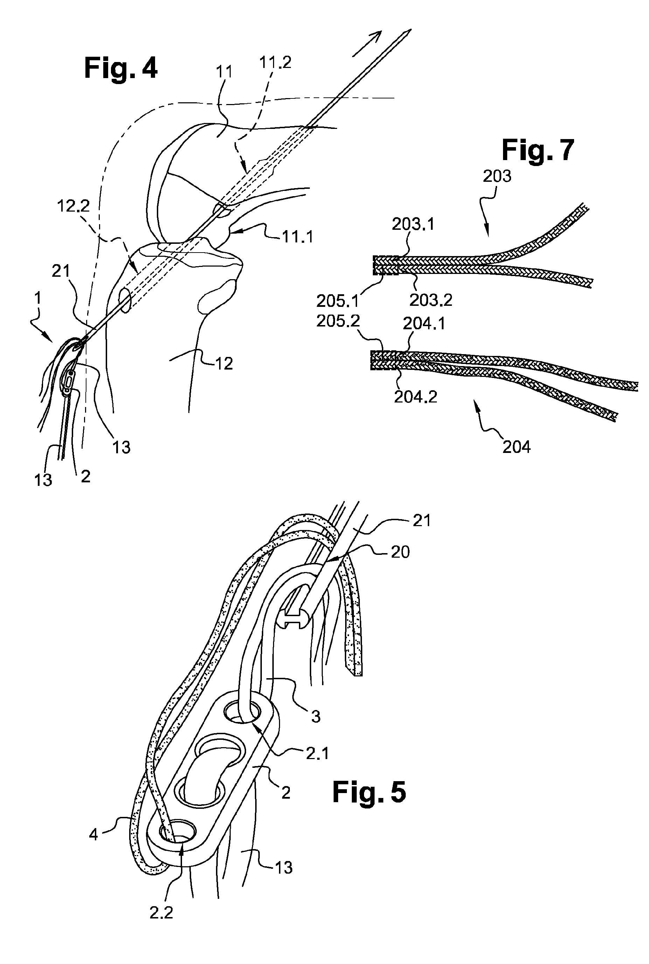

Comme le montre la figure 2, l'ensemble de fixation 1 comprend une platine 2 composée de titane. La platine 2 forme un élément de fixation adapté pour fixer sur une surface osseuse une partie du greffon orthopédique. Dans l'exemple des figures 1 à 5, la platine 2 est destinée à fixer un greffon de ligament croisé antérieur, comme cela est décrit ci-après en relation avec les figures 4 et 5.

En outre, l'ensemble de fixation 1 comprend deux fils 3 et 4 qui présentent chacun au moins deux portions d'extrémité. Comme le montre la figure 3, le fil 3 et le fil 4 présentent respectivement deux portions d'extrémité 3.1 et 3.2 et deux portions d'extrémité 4.1 et 4.2. Chaque fil 3 ou 4 a un diamètre d'environ 0,85 mm.

Chaque fil 3 ou 4 est lié à la platine 2 de sorte qu'une traction exercée sur le fil respectif 3 ou 4 réalise un mouvement respectif de la platine 2. 1. En service, après que l'ensemble de fixation 1 muni du greffon a traversé un passage osseux, tel qu'illustré à la figure 4, une traction sur le fil 3 permet de réaliser une translation de la platine 2, substantiellement suivant la direction du passage osseux, tandis qu'une traction sur le fil 4 permet de réaliser un pivotement de la platine 2 pour la positionner sur la corticale du fémur.

Les fils 3 et 4 ont de préférence des couleurs différentes, pour faciliter leur repérage par un opérateur les manipulant. Par exemple, le fil 2 peut être blanc et le fil 4 peut être vert.

Comme le montre la figure 3, les quatre portions d'extrémité 3.1, 3.2, 4.1 et 4.2 sont solidarisées ensemble au moyen d'une soudure 5 formée par un matériau plastique de synthèse. En l'occurrence, le matériau plastique de synthèse est un polyester. Chaque fil 3 ou 4 est composé totalement de ce polyester.

La soudure 5 peut être réalisée par tout procédé de soudage, c'est-à-dire au moyen d'un échauffement du matériau plastique de synthèse au-dessus de sa température de ramollissement ou de fusion. Par exemple, la soudure 5 peut être réalisée par soudage thermique par effet Joule, aux ultrasons, par laser ou par hautes fréquences. Alternativement, la soudure 5 peut être réalisée sans apport thermique par un procédé de soudure chimique à froid au moyen de solvants.

Les portions d'extrémité 3.1, 3.2, 4.1 et 4.2 sont solidarisées, par la soudure 5, sur une longueur L5 d'au moins 1,0 mm et de préférence sur une longueur de 3,0 mm. La longueur L5 est mesurée suivant la direction longitudinale d'un fil 3 ou 4. De plus, les quatre portions d'extrémité 3.1, 3.2, 4.1 et 4.2 sont deux à deux juxtaposées de sorte qu'elles forment un faisceau plat, ce qui permet le passage du faisceau plat dans un chas étroit et oblong. Dans l'exemple des figures, ce faisceau plat a une épaisseur inférieure à 1 mm et une largeur inférieure à 6 mm.

Les portions d'extrémité 3.1, 3.2, 4.1 et 4.2 sont substantiellement parallèles et ont des parties terminales respectives 3.3, 3.4, 4.3 et 4.4 qui sont adjacentes. Les parties terminales 3.3, 3.4, 4.3 et 4.4 des portions d'extrémité 3.1, 3.2, 4.1 et 4.2 sont solidarisées entre elles par la soudure 5.

Pour lier chaque fil 3 ou 4 à la platine 2, la platine 2 présente deux trous distants 2.1 et 2.2. Chaque fil 3 ou 4 passe dans un trou respectif 2.1 ou 2.2 de façon à former, avec les portions d'extrémité correspondantes 3.1, 3.2, 4.1 et 4.2, une boucle liant le fil 3 ou 4 à la platine 2.

Chaque portion d'extrémité 3.1, 3.2, 4.1 ou 4.2 présente une dureté superficielle supérieure à la dureté du reste du fil respectif 3 ou 4, afin de faciliter sa préhension et son soudage aux autres portions d'extrémité.

La soudure 5 est conçue pour présenter un effort de séparation de deux portions d'extrémité respectives 3.1 et 3.2 ou 4.1 et 4.2 qui est inférieur à 8 newtons, ce qui permet une séparation manuelle de ces portions d'extrémité. Cet effort de séparation est mesuré par exemple en tirant sur deux portions d'extrémité 4.1 et 4.2 dans des sens opposés et suivant une direction transversale Y4 perpendiculaire à la direction longitudinale X4 du fil 4.

La soudure 5 est conçue pour résister à un effort de séparation de deux portions d'extrémité respectives 3.1 et 3.2 ou 4.1 et 4.2 qui est supérieur à 1,0 newtons, ce qui permet d'éviter une séparation intempestive et inopinée de ces portions d'extrémité.

Les figures 4 à 5 illustre une étape d'un procédé d'installation de l'ensemble de fixation 1 sur une articulation de genou, entre un fémur 11 et un tibia 12. Le greffon orthopédique 13 est ici formé par un substitut de ligament croisé antérieur et la surface osseuse est la surface corticale 11.1 du fémur 11 pour lequel il faut remplacer le ligament croisé antérieur. Le greffon orthopédique 13 a une forme allongée.

Le greffon orthopédique 13 peut être composé de fibres tissulaires ou synthétiques. Le greffon orthopédique 13 est destiné à traverser deux tunnels ou passages osseux 11.2 et 12.2, percés respectivement dans le fémur 11 et dans le tibia 12. Les passages osseux 11.2 et 12.2 sont rectilignes et colinéaires.

L'ensemble de fixation 1 comprend en outre une cordelette 6 qui est liée à la platine 2 et à laquelle est attaché le greffon orthopédique 3.

Les fils sont passés dans un chas 20 d'une broche 21, laquelle a notamment pour fonction de guider l'ensemble de fixation 1 et le greffon orthopédique 13 à travers les passages osseux 11.2 et 13.2.

Dans la mesure où le faisceau des quatre portions d'extrémité 3.1, 3.2, 4.1 et 4.2 est plat, les fils 3 et 4 peuvent être enfilés à travers le chas 20 qui est étroit et oblong. Dans l'exemple des figures 4 et 5, le chas 20 mesure environ 1 mm de large sur environ 6 mm de long.

Un procédé de fixation conforme à l'invention, pour fixer sur la surface corticale 11.1 le greffon orthopédique 13 via les passages osseux 11.2 et 12.2, comprend les étapes :
- mettre en oeuvre l'ensemble de fixation 1;
- lier chaque fil 3 ou 4 à la platine 2 de sorte qu'une traction exercée sur un fil respectif 3 ou 4 réalise un mouvement respectif de la platine 2 ; et
- solidariser les quatre portions d'extrémité 3.1, 3.2, 4.1 et 4.2 au moyen de la soudure 5.

La figure 6 illustre une partie d'un ensemble de fixation 101 conforme à un deuxième mode de réalisation de l'invention. Dans la mesure où l'ensemble de fixation 101 est similaire à l'ensemble de fixation 1, la description de l'ensemble de fixation 1 donnée ci-avant en relation avec les figures 1 à 5 peut être transposée à l'ensemble de fixation 101, à l'exception notable des différences énoncées ci-après.

Un élément de l'ensemble de fixation 101 identique ou correspondant, par sa structure ou par sa fonction, à un élément de l'ensemble de fixation 1 porte la même référence numérique augmentée de 100. On définit ainsi deux fils 103 et 104, une soudure 105, quatre portions d'extrémité 103.1, 103.2, 104.1 et 104.2

L'ensemble de fixation 101 diffère de l'ensemble de fixation 1, car il a quatre parties terminales 103.3, 103.4, 104.3 et 104.4 qui sont libres, c'est-à-dire exemptes de soudure. En d'autres termes, la soudure 105 est formée en amont de chacune des parties terminales 103.3, 103.4, 104.3 et 104.4. Chaque partie terminale 103.3, 103.4, 104.3 ou 104.4 a une longueur L104.4 qui est comprise entre 0,1 mm et 15,0 mm.

La figure 7 illustre une partie d'un ensemble de fixation 201 conforme à un deuxième mode de réalisation de l'invention. Dans la mesure où l'ensemble de fixation 201 est similaire à l'ensemble de fixation 1, la description de l'ensemble de fixation 1 donnée ci-avant en relation avec les figures 1 à 5 peut être transposée à l'ensemble de fixation 201, à l'exception notable des différences énoncées ci-après.

Un élément de l'ensemble de fixation 201 identique ou correspondant, par sa structure ou par sa fonction, à un élément de l'ensemble de fixation 1 porte la même référence numérique augmentée de 200. On définit ainsi deux fils 203 et 204, une soudure 205, quatre portions d'extrémité 203.1, 203.2, 204.1 et 204.2.

L'ensemble de fixation 201 diffère de l'ensemble de fixation 1, car les quatre portions d'extrémité 203.1, 203.2, 204.1 et 204.2 sont solidarisées en deux groupes de deux portions d'extrémité, 203.1 et 203.2 d'une part, 204.1 et 204.2 d'autre part, de façon à former deux faisceaux distincts.

Selon des caractéristiques avantageuses mais facultatives de l'invention, prises isolément ou selon toute combinaison techniquement possibles :
- Au lieu de solidariser par soudure les deux portions d'extrémité d'un même fil, deux portions d'extrémité de deux fils distincts sont soudées entre elles. En d'autres termes, les portions d'extrémité sont « croisées » pour former des faisceaux.
- Le matériau plastique de synthèse pour la soudure est présent uniquement au niveau des portions d'extrémités.
- Le matériau plastique de synthèse est un revêtement déposé sur chaque fil, l'âme de chaque fil étant composée d'un matériau distinct.
- Au lieu d'être tressé, chaque fil peut être un fil monobrin, c'est-à-dire à une seule fibre.
- Plusieurs soudures distinctes sont réalisées, pour réduire le risque de séparation intempestive des portions d'extrémité.

## Revendications

1. Ensemble de fixation (1), pour fixer sur une surface osseuse (11.1) un greffon orthopédique (13), l'ensemble de fixation (1) comprenant :
- au moins un élément de fixation (2) adapté pour fixer sur une surface osseuse (11.1) une partie du greffon orthopédique (13);
- au moins un fil (3, 4 ; 103, 104; 203, 204) qui présente au moins deux portions d'extrémité (3.1, 3.2, 4.1, 4.2 ; 103.1, 103.2, 104.1, 104.2; 203.1, 203.2, 204.1, 204.2) et qui est lié à l'élément de fixation (2) de sorte qu'une traction exercée sur ledit fil (3, 4 ; 103, 104; 203, 204) réalise un mouvement respectif de l'élément de fixation (2) ;
l'ensemble de fixation (1) étant **caractérisé en ce qu'**au moins deux desdites portions d'extrémité (3.1, 3.2, 4.1, 4.2; 103.1, 103.2, 104.1, 104.2; 203.1, 203.2, 204.1, 204.2) sont solidarisées au moyen d'au moins une soudure (5 ; 105; 205) formée par au moins un matériau plastique de synthèse.

2. Ensemble de fixation (1) selon la revendication 1, dans lequel ledit au moins un fil (3, 4 ; 103, 104; 203, 204) est composé totalement ou partiellement par ledit matériau plastique de synthèse.

3. Ensemble de fixation (1) selon l'une des revendications précédentes, dans lequel le matériau plastique de synthèse est sélectionné dans le groupe constitué du polyester, polypropylène, polyéthylène haute ténacité, polyamide, PLLA, ou copolymère d'acide lactique et glycolique.

4. Ensemble de fixation (1) selon l'une des revendications précédentes, dans lequel lesdites au moins deux portions d'extrémité (3.1, 3.2, 4.1, 4.2 ; 103.1, 103.2, 104.1, 104.2; 203.1, 203.2, 204.1, 204.2) sont substantiellement parallèles et ont des parties terminales (3.3, 3.4, 4.3, 4.4 ; 103.3, 103.4, 104.3, 104.4) adjacentes.

5. Ensemble de fixation (1) selon l'une des revendications précédentes, comprenant au moins deux fils (3, 4) et dans lequel chacun desdits fils (3, 4) est lié à l'élément de fixation (2) de sorte qu'une traction exercée sur un fil (3, 4) respectif réalise un mouvement respectif de l'élément de fixation (2), et dans lequel au moins quatre portions d'extrémité (3.1, 3.2, 4.1, 4.2) sont solidarisées ensemble.

6. Ensemble de fixation (1) selon la revendication 5, dans lequel lesdites au moins quatre portions d'extrémité (3.1, 3.2, 4.1, 4.2) sont deux à deux juxtaposées de sorte qu'elles forment un faisceau plat, le faisceau plat ayant de préférence une épaisseur inférieure à 1 mm et une largeur inférieure à 6 mm.

7. Ensemble de fixation (1) selon l'une des revendications 5 ou 6, dans lequel au moins deux fils (3, 4; 103, 104; 203, 204) ont des couleurs différentes, par exemple un fil blanc (3 ; 103; 203) et un fil vert (4 ; 104; 204).

8. Ensemble de fixation (1) selon l'une des revendications précédentes, dans lequel ladite au moins une soudure (5 ; 105; 205) est conçue pour présenter un effort de séparation de deux portions d'extrémité (3.1, 3.2, 4.1, 4.2; 103.1, 103.2, 104.1, 104.2; 203.1, 203.2, 204.1, 204.2) qui est inférieur à 12 newtons, de préférence à 8 newtons, de préférence encore à 6 newtons.

9. Ensemble de fixation (1) selon l'une des revendications précédentes, dans lequel ladite au moins une soudure (5; 105; 205) est conçue pour résister à un effort de séparation de deux portions d'extrémité (3.1, 3.2, 4.1, 4.2; 103.1, 103.2, 104.1, 104.2; 203.1, 203.2, 204.1, 204.2) qui est supérieur à 0,8 newtons, de préférence à 1,0 newtons.

10. Ensemble de fixation (1) selon l'une des revendications précédentes, dans lequel ladite au moins une soudure (5 ; 105; 205) est réalisée par soudage thermique par effet Joule, par laser, par haute fréquence ou par ultrasons ou par soudage à froid au moyen de solvants.

11. Ensemble de fixation (1) selon l'une des revendications précédentes, dans lequel lesdites au moins deux portions d'extrémité (3.1, 3.2, 4.1, 4.2) sont solidarisées sur une longueur supérieure à 1 mm, de préférence à 3 mm.

12. Ensemble de fixation selon l'une des revendications précédentes, dans lequel au moins deux portions d'extrémité (103.1, 103.2, 104.1, 104.2) respectives présentent des parties terminales (103.3, 103.4, 104.3, 104.4) qui sont exemptes de soudure (105) et qui ont de préférence une longueur (L104.3) comprise entre 0,1 mm et 15,0 mm, ladite au moins une soudure (105) étant formée en amont de chacune desdites parties terminales (103.3, 103.4, 104.3, 104.4).

13. Ensemble de fixation (1) selon l'une des revendications 1 à 11, dans lequel au moins deux portions d'extrémité (3.1, 3.2, 4.1, 4.2 ; 203.1, 203.2, 204.1, 204.2) respectives présentent des parties terminales (3.3, 3.4, 4.3, 4.4) solidarisées par ladite au moins une soudure (5 ; 205).

14. Ensemble de fixation (1) selon l'une des revendications précédentes, dans lequel l'élément de fixation (2) présente deux trous (2.1, 2.2) distants, le ou chaque fil (3, 4) passant dans un trou de façon à former, avec les portions d'extrémité (3.1, 3.2, 4.1, 4.2) correspondantes, une boucle liant ledit fil (3, 4) à l'élément de fixation (2), l'élément de fixation (2) étant par exemple une platine destinée à fixer un greffon orthopédique (13) formant un ligament croisé.

15. Procédé de fixation, pour fixer sur une surface osseuse (11.1) un greffon orthopédique (13) qui est destiné à traverser au moins un passage osseux (11.2, 12.2), le procédé de fixation comprenant les étapes :
- mettre en oeuvre un ensemble de fixation (1) selon l'une des revendications précédentes;
- lier ledit au moins un fil (3, 4; 103, 104; 203, 204) à l'élément de fixation (2) de sorte qu'une traction exercée sur un fil (3, 4 ; 103, 104; 203, 204) respectif réalise un mouvement respectif de l'élément de fixation (2) ; et
- solidariser au moins deux desdites portions d'extrémité (3.1, 3.2, 4.1, 4.2; 103.1, 103.2, 104.1, 104.2; 203.1, 203.2, 204.1, 204.2) au moyen d'au moins une soudure (5 ; 105; 205) formée par au moins un matériau plastique de synthèse, ladite au moins une soudure (5 ; 105 ; 205) étant est réalisée par un procédé de soudage sélectionné dans le groupe constitué du soudage thermique par effet Joule, par laser, par haute fréquence ou par ultrasons et du soudage à froid au moyen de solvants.
